(19) Europäisches Patentamt
European Patent Office
Office européen des brevets

(11) Publication number: **0 298 431 B1**

(12) **EUROPEAN PATENT SPECIFICATION**

(45) Date of publication of patent specification: **29.01.92**

(51) Int. Cl.5: **C07C 53/128**, C07C 51/14, C07C 51/44, C07C 51/48

(21) Application number: **88110732.0**

(22) Date of filing: **05.07.88**

(54) **Process for producing carboxylic acid.**

(30) Priority: **07.07.87 JP 169099/87**
**06.07.87 JP 168327/87**

(43) Date of publication of application:
**11.01.89 Bulletin 89/02**

(45) Publication of the grant of the patent:
**29.01.92 Bulletin 92/05**

(84) Designated Contracting States:
**BE CH DE FR GB IT LI NL SE**

(56) References cited:
**GB-A- 1 016 087**
**US-A- 3 059 006**
**US-A- 3 910 963**

**PATENT ABSTRACTS OF JAPAN, vol. 10, no. 245 (C-368)[2301], 22nd August 1986; & JP-A-61 76 434**

**CHEMICAL ABSTRACTS, vol. 84, no. 7, 16th February 1976, page 43336, abstract no. 43333n, Columbus, Ohio, US; & JP-A-75 123614**

(73) Proprietor: **IDEMITSU PETROCHEMICAL CO. LTD.**
**1-1, Marunouchi 3-chome Chiyoda-ku**
**Tokyo 100(JP)**

(72) Inventor: **Kawasaki, Hiroshi Idemitsu Petrochemical Comp. Ltd**
**1-1, Miyame-machi**
**Tokuyama-shi Yamaguchi-ken(JP)**

(74) Representative: **TER MEER - MÜLLER - STEINMEISTER & PARTNER**
**Mauerkircherstrasse 45**
**W-8000 München 80(DE)**

**EP 0 298 431 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention relates to a novel process for producing a carboxylic acid according to which a carboxylic acid of high purity can be produced and besides, sulfuric acid can be effectively separated from reaction mixture of Koch's reaction which uses a catalyst containing sulfuric acid and this can be reused.

Description of the Related Art

Koch's reaction (cf. Japanese Patent Kokoku No.30-3362) relates to a process for producing a carboxylic acid by contacting an olefin and an alcohol with carbon monoxide and water using an inorganic strongly acidic catalyst. Industrially and practically valuable olefins used in this process are monoolefins of 3-20 carbon atoms. The inorganic strongly acidic catalysts include, for example, sulfuric acid, phosphoric acid and sulfuric acid, boron trifluoride and water, hydrogen fluoride, phosphoric acid boron trifluoride complex and sulfuric acid boron trifluoride complex. Among them, sulfuric acid is industrially widely used because it is inexpensive as compared with other catalyst components such as boron trifluoride, is easily available and is good in reaction yield.

However, crude carboxylic acids prepared by Koch's reaction using catalysts containing sulfuric acid contain sulfur compounds such as sulfites and sulfates and furthermore, the resulting crude carboxylic acids also contain a slight amount of sulfuric acid. Thus, there is the problem of coloration. Further, these sulfuric acid and sulfur compounds contained cannot be completely removed by operation such as washing. Therefore, carboxylic acids produced by using catalysts containing sulfuric acid suffer from the problems that purity decreases due to contamination with sulfur, the carboxylic acids are colored and are readily decomposed. Further problems are that reaction mixture is carbonized when carboxylic acid is distilled and apparatus is corroded with sulfur compounds such as sulfuric acid contained in the resulting carboxylic acid.

Under the circumstances, a number of methods to purify crude carboxylic acids obtained by Koch's reaction have been proposed.

For example, one of them is to distil crude carboxylic acids under normal pressure or reduced pressure. However, this method requires a pretreatment to remove sulfur compounds or a slight amount of sulfuric acid since a considerable amount of carboxylic acids are decomposed with the sulfur compounds or a slight amount of the sulfuric acid at distillation.

The pretreatment includes an alkali extraction method which comprises contacting crude carboxylic acid with an aqueous solution of a caustic alkali to selectively convert the carboxylic acid to alkali salt, separating this salt in the form of an aqueous solution and adding a mineral acid to this aqueous solution to separate the carboxylic acid. However, this method requires a large amount of inorganic compounds and in addition, inorganic substances incorporate at the acid separation, resulting in reduction of purity.

Japanese Patent Kokoku No.38-11964 discloses a process which comprises contacting a crude carboxylic acid with an aqueous ammonia solution to obtain an aqueous solution of ammonium salt of carboxylic acid and heating this aqueous solution to a temperature higher than decomposition temperature of the ammonium salt to eliminate ammonia and to obtain the objective carboxylic acid. However, this process suffers from the problem that a considerable amount of carboxylic acid is decomposed at the decomposition by heating.

There are further processes, one of which comprises contacting a crude carboxylic acid with a complex compound forming agent to form a complex of metal compound contained in the carboxylic acid and then washing the acid with an alkali (Japanese Patent Kokoku No.38-14917) and another of which comprises contacting a crude carboxylic acid with an oxidizing agent prior to distillation of the crude carboxylic acid (Japanese Patent Kokoku No.48-35048). However, these processes aim at mainly separation of the objective carboxylic acid (branched carboxylic acid) from polymerized oil which is oligomer of starting olefin, inactivation of metal compound which promotes discoloration and decomposition of the branched carboxylic acid and decoloration of crude carboxylic acid. Therefore, these processes are somehow effective for improvement of properties of crude carboxylic acid, but are not so effective for prevention of corrosion, reduction of purity, coloration, decomposition and carbonization of residual oils which are caused by sulfites and sulfates contained in the crude carboxylic acid or a slight amount of sulfuric acid produced by decomposition of sulfur compounds.

Further, a process is known which comprises heating a crude carboxylic acid together with water or a small amount of inorganic salt under liquid phase conditions of temperature 100-270 °C prior to distillation of the crude carboxylic acid (Japanese Patent Kokoku No.48-35048).

However, this process requires additional step of heating the crude carboxylic acid together with water

or a small amount of inorganic salt to 100-270 °C and so, for practice of this step, heat treating apparatus and apparatus for removal of aqueous layer used in the heat treatment must be added to the conventional apparatus. Further, if salts of carboxylic acid such as formic acid, acetic acid, propionic acid, oxalic acid or phthalic acid are used as the inorganic salts, this carboxylic acid may incorporate into the objective carboxylic acid (branched carboxylic acid), resulting in reduction of purity of the resulting carboxylic acid.

As explained above, the conventional processes are not suitable for producing carboxylic acids of high purity.

On the other hand, Koch's reaction requires use of at least 3 mols of sulfuric acid for 1 mol of olefin which is a starting material. Thus, since amount of catalyst used is large, it is an important point in industrial production of carboxylic acid utilizing Koch's reaction to recover sulfuric acid used as a catalyst after the reaction and effectively reuse it as catalyst.

Especially, carboxylic acids of 4-9 carbon atoms among carboxylic acids produced by Koch's reaction show very high solubility in sulfuric acid. Therefore, it is important for not only reuse of sulfuric acid, but also for improvement of reaction yield to efficiently separate the carboxylic acid from a large amount of sulfuric acid without causing reduction of concentration of sulfuric acid. Especially, pivalic acid which is a carboxylic acid having 5 carbon atoms and which is very useful as starting material for agricultural agents and reaction initiators is especially high in solubility in sulfuric acid and development of a process for efficient production without reduction of sulfuric acid concentration has been demanded.

As a process for production of carboxylic acid utilizing Koch's reaction, Japanese Patent Kokai No.42-12402 discloses a process according to which water is added to a reaction product containing carboxylic acid and sulfuric acid catalyst to liberate carboxylic acid or dissolve the carboxylic acid in water and thereafter, when carboxylic acid is liberated, this is obtained by decantation and when it is dissolved in water, carboxylic acid is extracted with a saturated hydrocarbon such as pentane, hexane or heptane.

However, in this process, the sulfuric acid catalyst must be diluted with a large amount of water to separate the carboxylic acid. As a result, in order to reuse sulfuric acid as a catalyst, a large amount of the dilute sulfuric acid obtained must be concentrated and so this process is not industrial from the aspect of energy. Further, by-products in Koch's reaction such as sulfates and sulfites contained in the dilute sulfuric acid are sometimes decomposed at the concentrating to produce sulfur dioxide. Furthermore, since these compounds are carbonized or become tar with concentrating of sulfuric acid, sulfuric acid sometimes cannot be concentrated to the extent where the sulfuric acid can be used as a catalyst (sulfuric acid concentration must be 80% by weight or more in order that it can be used for Koch's reaction).

Japanese Patent Kokoku No.48-18216 discloses a process according to which water in an amount substantially equimolar to carbon monoxide consumed in Koch's reaction is added to the reaction product containing carboxylic acid and sulfuric acid catalyst to migrate the carboxylic acid and sulfuric acid contained in the reaction product into the aqueous phase, then a halogenated hydrocarbon is added to the separated aqueous solution to migrate the carboxylic acid into the halogenated hydrocarbon phase, then this halogenated hydrocarbon phase is separated, whereby sulfuric acid of theoretically the same concentration as of the sulfuric acid used is obtained from the aqueous phase and carboxylic acid is obtained from the halogenated hydrocarbon phase.

However, since considerable amount of the carboxylic acid in the halogenated hydrocarbon is present in the form of a complex with sulfuric acid in the halogenated hydrocarbon, concentration of the recovered sulfuric acid is reduced in correspondence to the amount of the sulfuric acid which forms a complex with carboxylic acid. Therefore, when sulfuric acid recovered by this process is reused as a catalyst, concentration of sulfuric acid decreases with increase in the number of use and hence, sufficient number of repetition cannot be secured.

## SUMMARY OF THE INVENTION

An object of this invention is to provide a process for production of carboxylic acid of high purity by effective removal of sulfur-containing compounds such as sulfites, sulfates and sulfuric acid which are contained in crude carboxylic acid obtained by reacting an olefin, carbon monoxide and water in the presence of a catalyst containing sulfuric acid.

Another object of this invention is to provide a process for production of carboxylic acid according to which reduction of purity, coloration and decomposition of crude carboxylic acid obtained by Koch's reaction can be prevented and besides, carbonization of residual oils in the production of the crude carboxylic acid and corrosion of apparatuses when the resulting carboxylic acid is used can be prevented.

Still another object of this invention is to provide a process for production of high purity carboxylic acid by reacting an olefin, carbon monoxide and water in the presence of a catalyst containing sulfuric acid

wherein the sulfuric acid catalyst used can be effectively recovered and circulatingly used.

This invention for attaining the above objects is a process for producing a carboxylic acid, characterized by including a reaction step of reacting an olefin, carbon monoxide and water in the presence of a catalyst containing sulfuric acid, an extraction step of extracting the carboxylic acid in the reaction mixture obtained in the above reaction step into an organic solvent and a distillation step of distilling the organic phase obtained in the above extraction step in the presence of at least one metal salt of carboxylic acid selected from the group consisting of alkali metal salts of carboxylic acid and alkaline earth metal salts of carboxylic acid.

Furthermore, this invention is a process for production of carboxylic acid, characterized by including a reaction step of reacting an olefin, carbon monoxide and water in the presence of a catalyst containing sulfuric acid; and extraction step comprising a step of contacting a reaction mixture obtained in the above reaction step with an organic solvent to separate into an aqueous phase and an organic phase and a step of contacting the resulting organic phase with water in an amount of not more than that of water consumed in the above reaction step to separate into a sulfuric acid-extraction phase and a carboxylic acid-extraction phase; a distillation step of distilling the carboxylic acid-extraction phase obtained in the above extraction step in the presence of at least one metal salt of carboxylic acid selected from the group consisting of alkali metal salts of carboxylic acid and alkaline earth metal salts of carboxylic acid; and a step of combining the aqueous phase and the sulfuric acid-extraction phase obtained in the above extraction step and recycling the mixture as a catalyst containing sulfuric acid in the above reaction step.

DESCRIPTION OF THE PREFERRED EMBODIMENT

The reaction step in this invention is a step of contacting an olefin with carbon monoxide and water in the presence of a catalyst containing sulfuric acid. The reaction in this step is known as Koch's reaction.

The olefins used in this invention include, for example, olefins and alcohols.

The olefins may have a double bond at any position in the molecule. As examples of such olefins, mention may be made of straight chain or branched chain olefins such as ethylene, propylene, n-butylene, isobutylene, diisobutylene, 1-hexene, 2-hexene, 3-hexene, 1-heptene, 2-heptene, 3-heptene, 1-octene, 2-octene, 3-octene, 4-octene, 1-nonene, 2-nonene, 3-nonene, 4-nonene and hexadecene and cycloolefins such as cyclohexene which may be substituted with alkyl group such as methyl and ethyl or vinyl group. Further examples are diolefins such as butadiene, acryl chloride and methacryl chloride.

Furthermore, in this invention, there may be used isobutylene oligomers such as diisobutylene and triisobutylene. The isobutylene oligomers are oligomers obtained by polymerization of about 2-12 isobutylene molecules in the presence of, for example, Lewis acid catalysts such as hydrochloric acid, sulfuric acid and boron trifluoride and normally have a double bond at terminal. They may be isomerized to internal olefins by gradual intramolecular rearrangement of the double bond due to catalytic action.

Examples of the isobutylene oligomers which can be used in this invention are shown below.

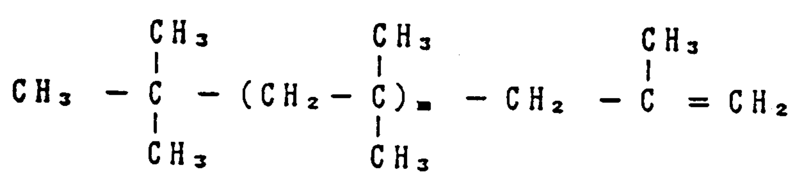

wherein m denotes an integer of 0-10.

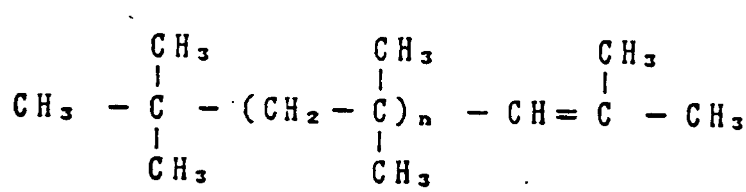

wherein n denotes an integer of 0-10.

The process of this invention is suitable for production of carboxylic acids having branched chain using

olefins having branched chain such as isobutylene and especially suitable for production of branched carboxylic acid of 7 or less carbon atoms such as pivalic acid.

In the reaction step of this invention, alcohols can be used as olefins in place of the above exemplified olefins.

The alcohols in this case are, for example those which have 2-22 carbon atoms and are of straight chain or of branched chain.

In the first step of this invention, in addition to the above mentioned olefins and alcohols, other compounds ordinarily used in Koch's reaction can also be used as the olefins.

The olefins can be used alone or in combination of two or more in the first step. Further, it is not needed to use only one purified olefin, but normally, mixtures of two or more olefins or mixtures of isomers are used. However, it is preferred for improvement of purity of the resulting carboxylic acids to use purified olefins. For example, in order to produce pivalic acid at high purity, it is effective to increase the concentration of isobutylene to 90% by weight or more (preferably 95% by weight or more) and to decrease content of n-butene. In this way, production of carboxylic acids of 9 carbon atoms derived from polymers of n-butene and isobutene can be effectively controlled.

In this invention, pure carbon monoxide is most preferred, but there may also be used carbon monoxide-containing gases obtained from water gas, generator gas and coke oven gas.

Pure water is most preferably used and distilled water and deionized water are normally used. However, tap water and industrial water may also be used since presence of chlorine compounds or metal compounds in a small amount does not prevent proceeding of Koch's reaction. Furthermore, as mentioned hereinafter, water used for adjustment of concentration of sulfuric acid used as a catalyst can also be used as a raw material instead of directly adding water to the reaction system.

The catalyst used in this invention is one containing sulfuric acid. Concentration of the sulfuric acid which is a catalyst in this invention is usually 80-90% by weight (preferably 82-88% by weight). If sulfuric acid of less than 80% by weight in concentration is used, Koch's reaction may not proceed smoothly and if that of more than 90% by weight is used, extraction rate of carboxylic acid at the extraction step sometimes decreases.

In this invention, the sulfuric acid is normally used alone. However, it is also possible to use other inorganic strongly acidic catalyst together with sulfuric acid. The other inorganic strongly acidic catalysts include, for example, combination of sulfuric acid and phosphoric acid, combination of boron trifluoride and water, hydrogen fluoride, phosphoric acid boron trifluoride complex and sulfuric acid boron trifluoride complex.

When Koch's reaction is effected using combination of a plurality of catalyst components including sulfuric acid, content of sulfuric acid in the catalyst, for example, content of sulfuric acid in the catalyst when a catalyst comprising sulfuric acid and phosphoric acid, is usually 30% by weight or more (preferably 40% by weight or more). When content of sulfuric acid is less than 30% by weight, sometimes Koch's reaction does not proceed smoothly.

Further, metallic compounds may also be used together with the inorganic strongly acidic catalysts such as sulfuric acid. Such metallic compounds include, for example, metal oxides such as cuprous oxide, silver oxide, silver sulfate and gold oxide, metals such as metallic copper and metal mixtures such as a mixture of divalent copper compound and metallic copper. When such metallic compound is used, cuprous oxide or silver oxide is preferred.

It is desirable to adjust contents of the components so that acid strength (Hammett's acidity function Ho) of catalyst containing sulfuric acid is within the range of -9.2 - -6 (preferably -9.0 - -6.5). When acid strength of the catalyst is higher than -6, the tendency of occurring of polymerization or isomerization of olefins increases and when less than -9.2, carboxylation reaction readily occurs, but there is the tendency of reduction in extraction rate of carboxylic acid from catalyst.

Method for preparation of the initial catalyst used in the reaction step of this invention is not critical and usual methods can be employed.

The reaction step of this invention can be performed by any of batch process, semi-batch process and continuous process.

Other reaction conditions can be ordinary ones.

For example, reaction temperature is normally 0-50°C (preferably 15-40°C) considering reaction velocity and amount of sulfuric acid sludge produced.

Reaction pressure is normally set within the range of 0-14,7 MPa G (preferably 0.196-98 MPa (G). This reaction pressure is usually adjusted by controlling the introduction pressure of carbon monoxide introduced into reaction vessel.

The olefin which is a starting material is preferably gradually fed to the reaction vessel over a period of

0.5-5 hours (preferably 1-4 hours). Feeding of this olefin is normally carried out with stirring. If the olefin is fed in a short time, polymerization reaction sometimes occurs predominantly.

The reaction may be discontinued immediately after feeding of olefin, but normally stirring is carried out for further 3 hours or less (preferably 2 hours) after the feeding of olefin to allow the reaction to proceed.

The extraction step of this invention is a step to extract a carboxylic acid in the reaction mixture obtained in the preceding reaction step into an organic solvent.

In Koch's reaction using a catalyst containing sulfuric acid, the sulfuric acid used as catalyst is mostly present as sulfuric acid in the reaction mixture and partially forms a complex with carboxylic acid. Therefore, an organic solvent is added to the reaction mixture to migrate the carboxylic acid and the complex of carboxylic acid and sulfuric acid into the organic solvent, thereby to separate and recover the sulfuric acid used as a catalyst while purifying the carboxylic acid.

The separated organic solvent phase contains carboxylic acid and complex of carboxylic acid and sulfuric acid. When pivalic acid is extracted with dichloromethane, said complex of carboxylic acid and sulfuric acid is formed with 1 mol of pivalic acid and 0.4-0.5 mol of sulfuric acid and when a branched carboxylic acid of 9 carbon atoms is extracted with n-hexane, the complex is formed with 1 mol of carboxylic acid and 0.2-0.25 mol of sulfuric acid.

The organic solvents used here include, for example, aliphatic hydrocarbons, aromatic hydrocarbons and aliphatic or aromatic hydrocarbons of 1-20 carbon atoms having at least one halogen atom as a substituent. These are preferably those which are low in dissolvability for water and high in dissolvability for carboxylic acid and can effectively extract carboxylic acid.

As examples of such organic solvents, mention may be made of aliphatic hydrocarbons such as n-pentane, n-hexane, n-heptane and n-octane, aromatic hydrocarbons such as benzene, toluene and xylene, monohalogenated aliphatic hydrocarbons such as monohalogenated methyl, monohalogenated ethyl, monohalogenated propyl, monohalogenated butyl and monohalogenated amyl, dihalogenated aliphatic hydrocarbons such as dihalogenated methylene, dihalogenated ethylene, dihalogenated ethylidene, dihalogenated trimethylene, dihalogenated propylidene, dihalogenated propyl, dihalogenated tetramethylene, dihalogenated butylidene and dihalogenated butylene, polyhalogenated aliphatic hydrocarbons such as haloforms, carbon tetrachloride, trihalogenated ethane, trihalogenated ethylene, tetrahalogenated ethane, pentahalogenated ethane and hexahalogenated ethane, monohalogenated aromatic hydrocarbons such as monohalogenated benzene, monohalogenated toluene and monohalogenated xylene, dihalogenated aromatic hydrocarbons such as dihalogenated benzene, dihalogenated toluene and dihalogenated xylene and polyhalogenated aromatic hydrocarbons such as trihalogenated benzene, benzotrihalide, trihalogenated toluene and xylene trihalide. The halogen atoms here include fluorine atom, chlorine atom, iodine atom, etc. and chlorine atom is preferred in this invention.

It is especially preferred to use one halogenated hydrocarbon selected from dichloromethane, chloroform and trichloroethylene or a mixture thereof.

One reason why halogenated hydrocarbons are preferred is that halogenated hydrocarbons have high extracting ability of carboxylic acid obtained by Koch's reaction. By using the halogenated hydrocarbons, it becomes possible to effectively extract such carboxylic acids (including complex compounds of such carboxylic acids and sulfuric acid) as pivalic acid which have less carbon atoms, are low in extractability with saturated aliphatic hydrocarbons or aromatic hydrocarbons and have high solubility in sulfuric acid.

Amount of organic solvents, especially halogenated hydrocarbons, added to the reaction mixture can be suitably set considering the solubility of carboxylic acid. However, taking into consideration the extractability of carboxylic acid and the efficiency for removal of the halogenated hydrocarbon when obtaining the carboxylic acid, content of halogenated hydrocarbon per total amount of the reaction mixture and the halogenated hydrocarbon is preferably 30-70% by weight (more preferably 40-60% by weight). When less than 30% by weight, extraction of carboxylic acid in the catalyst phase is sometimes not sufficiently high and even when more than 70% by weight of the halogenated hydrocarbon is used, extractability is no longer increased so much.

Other kind of solvents can be used in combination with the halogenated hydrocarbons as far as extracting ability of halogenated hydrocarbons is not damaged.

In this way, by using halogenated hydrocarbons, at least 90% by weight of carboxylic acid produced in the steady state at repeated use of catalyst is migrated to the halogenated hydrocarbon phase.

Halogenated hydrocarbon is added to the reaction mixture to migrate the reaction products such as carboxylic acid into halogenated hydrocarbon phase and thereafter this halogenated hydrocarbon phase is separated.

On the other hand, the phase from which the halogenated hydrocarbon phase has been separated contains mainly sulfuric acid used as a catalyst and this phase is used as catalyst for the next Koch's

reaction.

A more preferable extraction step in this invention comprises extracting the carboxylic acid in the reaction mixture obtained in the reaction step into an organic solvent resulting in separation into an aqueous phase and an organic phase and adding water to the separated organic phase to separate into a sulfuric acid extraction phase containing sulfuric acid and a carboxylic acid extraction phase containing carboxylic acid.

The addition of water to the organic phase is to decompose the complex formed with a part of sulfuric acid and carboxylic acid contained in the organic phase to extract sulfuric acid into aqueous phase and carboxylic acid into organic phase.

Amount of water added here is not more than the amount of water consumed by the reaction step.

This is because concentration of the recovered sulfuric acid must be maintained in order to use it in the reaction step.

That is, amount (A) of water to be added is shown by the following formula:

$$A\,(g) \leqq B \times \frac{C}{100} \times 18$$

wherein A represents amount of water to be added, B represents mol number of olefin used and C represents yield of carboxylic acid. As the yield of carboxylic acid, C, there may be used an average value obtained by productions of several times under same conditions.

It is especially preferred to set amount of water to be added so that sulfuric acid concentration in the generated catalyst is 80-90% by weight after combining the aqueous phase separated at this step with sulfuric acid extraction phase and furthermore after adding sulfuric acid in an amount corresponding to loss amount in respective operations.

Decomposition of the complex of carboxylic acid and sulfuric acid and migration of the sulfuric acid into aqueous phase can be performed by vigorously shaking the water and the organic solvent solution, preferably halogenated hydrocarbon solution in contact with each other. By this shaking, 60% by weight or more (preferably 75% by weight or more) of sulfuric acid component contained in the halogenated hydrocarbon phase is migrated to the aqueous phase.

The sulfuric acid extraction phase obtained by migration of sulfuric acid component into aqueous phase by the addition of water is separated from the carboxylic acid extraction phase containing carboxylic acid.

The thus obtained sulfuric acid extraction phase is reused as a catalyst in the next Koch's reaction as explained hereinafter and the carboxylic acid extraction phase is fed to the subsequent distillation step.

The distillation step in this invention is a step where the organic phase, preferably the carboxylic acid extraction phase obtained in the extraction step is distilled in the presence of at least one metal salt of carboxylic acid selected from the group consisting of alkali metal salts of carboxylic acid and alkaline earth metal salts of carboxylic acid.

In this distillation step, by allowing a metal salt of carboxylic acid to be present in the distillation system, a slight amount of impurities contained in the organic phase or carboxylic acid extraction phase such as reaction products of carboxylic acid and sulfuric acid, reaction products of carboxylic acid and sulfurous acid and sulfuric acid are trapped as alkali metal salts or alkaline earth metal salts to prevent incorporation of these impurities into carboxylic acid.

The alkali metal atoms which form the alkali metal salts of carboxylic acid include, for example, lithium atom, potassium atom and sodium atom and the alkaline earth metal atoms which form the alkaline earth metal salts of carboxylic acid include, for example, calcium atom and magnesium atom. The alkali metal atoms are preferred and sodium atom is especially preferred. Specifically, there may be used lithium hydroxide, potassium hydroxide, sodium hydroxide, calcium hydroxide and magnesium hydroxide.

There are no limitations in the method for allowing the alkali metal salt or alkaline earth metal salts of carboxylic acid to be present in distillation. For example there may be employed a method which comprises previously preparing an alkali metal salt or alkaline earth metal salt of carboxylic acid by reacting a carboxylic acid with an alkali metal compound or alkaline earth metal compound, adding this to the distillation system and then carrying out the distillation or a method which comprises adding the above mentioned hydroxide or the like before distillation to form alkali metal salt or alkaline earth metal salt of carboxylic acid in the system and then carrying out the distillation.

Amount of the alkali metal salt or alkaline earth metal salt of carboxylic acid to be present in distillation

is equal to or more than the equivalent amount of sulfur compounds such as the sulfuric acid compounds, sulfurous acid compounds and sulfuric acid contained in crude carboxylic acid. That is, this amount is not less than the amount which can neutralize the sulfur compounds. This is normally 1.0 equivalent or more (preferably 1.1-2.0 equivalents) per one equivalent of the sulfur compounds. By using such amount of the salt, the sulfur compounds contained in the crude carboxylic acid can be completely trapped and besides, even if the sulfur compounds or sulfurous compounds are decomposed by heating, the decomposition products can also be effectively trapped. Furthermore, reduction in the yield of carboxylic acid due to the addition of the alkali metal salt or alkaline earth metal salt of carboxylic acid is small.

Ordinarily, the organic solvent is removed from the organic phase containing carboxylic acid or the carboxylic acid extraction phase in the presence of the alkali metal salt or alkaline earth metal salt of carboxylic acid in the system.

After removal of the organic solvent, crude carboxylic acid is distilled in the presence of the alkali metal salt or alkaline earth metal salt of carboxylic acid.

The distillation can be performed either under normal pressure or reduced pressure.

Distillation temperature can be determined depending on the kind of carboxylic acid to be produced. For example a tertiary carboxylic acid of 9 carbon atoms can be obtained by distillation at a temperature within the range of 85-101 °C under a reduced pressure condition of 1 mmHg. A tertiary carboxylic acid of 13 carbon atoms can be obtained by distillation at a temperature within the range of 119-127 °C under the above reduced pressure condition. Furthermore, pivalic acid can be obtained by distillation at a temperature within the range of 163-164 °C under normal pressures.

Removal of the organic solvent and distillation of carboxylic acid may be carried out in succession.

Distillation of crude carboxylic acid can be carried out by ordinary distillation apparatus.

Sulfur content in the carboxylic acid thus purified by distillation in the presence of alkali metal salt or alkaline earth metal salt of carboxylic acid is usually 10 ppm or less. This content corresponds to about 1/6400 of sulfur content when distillation is carried out in the absence of alkali metal salt or alkaline earth metal salt. Therefore, the resulting carboxylic acid is neither colored nor smells bad.

Moreover, since distillation residue does not contain sulfuric acid, carbonization reaction does not proceed during distillation and the residue is not blackened due to carbonization.

In this invention, aqueous phase and sulfuric acid extraction phase obtained in the extraction step are combined and can be used as a catalyst containing sulfuric acid for the next reaction step.

That is, the aqueous phase separated in the extraction step contains sulfuric acid used as catalyst, unreacted olefins, carboxylic acids and the like. The sulfuric acid has higher concentration than before beginning of Koch's reaction because water is consumed.

Thus, by mixing the two phases, the aqueous phase containing sulfuric acid at high concentration is diluted with the sulfuric acid extraction phase. As a result of the dilution, concentration of sulfuric acid of the mixture is nearly the same as the concentration before the reaction.

Therefore, the thus obtained mixture can be used as a catalyst containing sulfuric acid for the next reaction step.

The mixture as such can be used as a catalyst for the reaction step, but since normally there is a loss of catalyst due to operation in organic reactions, it is desirable in this invention to add sulfuric acid to the mixture in an amount corresponding to the loss of catalyst caused by operation. This sulfuric acid added has an action of adjusting the sulfur concentration in the mixture to suitable ranges.

Recovery rate of sulfuric acid which can be recovered in this invention is generally at least 90% by weight (preferably at least 95% by weight). When the recovery is performed under more suitable conditions, recovery rate can be increased to higher than 99% by weight.

The thus obtained regenerated sulfuric acid catalyst has nearly the same catalytic activity as that of freshly prepared sulfuric acid and no conspicuous reduction of yield of carboxylic acid is seen even after continuous use of at least 40 times and further repetition is possible as far as the inventors have confirmed. Therefore, the resulting carboxylic acid is neither colored nor smells bad.

Further, since distillation residue contains no sulfuric acid, carbonization reaction does not proceed during the distillation and so the residue is not blackened due to carbonization of the residue.

According to the purification process of this invention, sulfur compounds contained in organic phase are trapped as alkali metal salt or alkaline earth metal salt by distillation of the organic phase obtained in the extraction step and therefore, carboxylic acid obtained by the distillation is not colored due to the sulfur compounds such as sulfuric acid and besides does not emit an offensive odor.

Moreover, decomposition of carboxylic acid due to the sulfur compounds in distillation can be effectively prevented and hence, the distillation residue is never carbonization.

Since content of sulfur compounds in carboxylic acid obtained by the process of this invention is very

low, corrosion of apparatuses which use this carboxylic acid can be effectively prevented.

In addition to the above explained excellent effects of the purification process of this invention, the process can be practised with conventional apparatuses as they are without complicated operations.

Furthermore, according to the process of this invention, catalyst containing sulfuric acid to be used in the next reaction step can be regenerated by combining aqueous phase and sulfuric acid extraction phase obtained in the extraction step.

This regenerated sulfuric acid catalyst is a regenerated product, but has the similar catalytic activity to that of freshly prepared catalyst and can be repeatedly used at least 40 times, which are markedly greater than those of sulfuric acids regenerated by conventional process.

Example 1

270 Grams of 85 wt% sulfuric acid was charged in a stainless steel autoclave of 1 liter in internal capacity and equipped with a magnetic stirrer and then the air in the reactor was fully replaced with carbon monoxide and further carbon monoxide was injected until the gage pressure reached 4.9 MPa G.

Then, 16.8 g of isobutylene was injected therein at 25 °C over a period of 3 hours under vigorous stirring, during which pressure in the autoclave was kept at 4.9 MPa G. After injection of the starting materials, stirring was continued for further 1 hour. (Reaction step).

After termination of the reaction, pressure was released and then, 200 ml of dichloromethane was introduced into the autoclave. Stirring was carried out for 1 minute, followed by leaving to stand for 10 minutes to separate the upper dichloromethane phase from the under aqueous phase.

To the separated dichloromethane extract was added 5.0 ml of water and this was vigorously shaken, thereby to recover 80% by weight of more of sulfuric acid which formed a complex as a dilute sulfuric acid. (Extraction step).

Composition of dichloromethane extract of pivalic acid is shown in the following Table 1.

Table 1

| Component | Composition |
|---|---|
| Dichloromethane | 90.0% by weight |
| Heavy oils | 0.06% by weight |
| Pivalic acid | 9.36% by weight |
| Heavy acids, etc. | 0.58% by weight |
| Total | 100.0% by weight |
| Sulfur content | 673 ppm by weight |

To 3000 g of a dichloromethane extract of crude pivalic acid obtained by the same operation as above was added 5.0 g of sodium hydroxide powder. Then, dichloromethane was distilled off to obtain about 305 g of crude pivalic acid. This fraction was distilled at room temperature by Widmer column of 1 cm in inner diameter and 75 cm in height. (Distillation step).

The initial fraction (10 g) was a mixture of water, a small amount of polymerized oil and pivalic acid. Subsequently, 260 g of purified pivalic acid having a sulfur content of 1 ppm and having a purity of 99.8% by weight was obtained at 163-164 °C.

Residual oil was a mixture of pivalic acid, heavy oils, heavy acids, sodium salts of carboxylic acid and sodium sulfate and had a weight of 35 g. No carbonization of the residual oil was recognized.

Dilute sulfuric acid obtained in the extraction step and said aqueous phase were combined and thereto was further added 2.8 g of 85 wt% sulfuric acid corresponding to loss amount due to reaction operation. Thus, catalyst to be used in the next reaction step was regenerated and the next reaction step was performed using this regenerated catalyst.

The above operation (reaction step - extraction step -distillation step) was repeated 40 times and yields of carboxylic acid and composition of the resulting liquid were measured and the results are shown in Table 2.

Table 2

| Number of reuse of catalyst (times) | Yield of carboxylic acid (mol %) | Composition of produced mixture (% by weight) | | |
|---|---|---|---|---|
| | | Heavy oils | Pivalic acid | Heavy acids |
| 1 | 51.4 | 0.0 | 96.7 | 3.3 |
| 2 | 99.4 | 0.0 | 93.9 | 6.1 |
| 3 | 73.2 | 0.0 | 95.0 | 5.0 |
| 4 | 88.9 | 0.0 | 95.9 | 4.1 |
| 5 | 94.4 | 0.5 | 95.1 | 4.4 |
| 6 | 106.1 | 0.9 | 94.8 | 4.3 |
| 7 | 102.7 | 0.0 | 95.0 | 5.0 |
| 8 | 104.5 | 0.4 | 95.3 | 4.3 |
| 9 | 97.5 | 0.0 | 96.1 | 3.9 |
| 10 | 99.8 | 0.5 | 95.1 | 4.4 |
| 11 | 98.1 | 0.0 | 95.6 | 4.4 |
| 12 | 97.3 | 1.1 | 93.8 | 5.1 |
| 13 | 98.8 | 0.0 | 95.2 | 4.8 |
| 14 | 94.9 | 0.7 | 95.1 | 4.2 |
| 15 | 92.3 | 1.0 | 93.8 | 5.2 |
| 16 | 99.2 | 1.1 | 95.2 | 3.7 |
| 17 | 99.6 | 1.1 | 93.8 | 5.1 |
| 18 | 93.7 | 0.0 | 92.8 | 7.2 |
| 19 | 95.8 | 0.6 | 90.2 | 9.2 |
| 20 | 98.6 | 0.0 | 92.2 | 7.8 |
| 21 | 94.8 | 0.0 | 93.4 | 6.4 |
| 22 | 93.1 | 0.9 | 95.6 | 3.5 |
| 23 | 94.9 | 0.5 | 96.0 | 3.5 |
| 24 | 96.8 | 0.0 | 95.6 | 4.4 |
| 25 | 96.6 | 1.1 | 95.3 | 3.6 |
| 26 | 95.8 | 0.6 | 96.2 | 3.2 |
| 27 | 95.2 | 2.5 | 95.8 | 1.7 |
| 28 | 94.5 | 0.7 | 95.2 | 4.1 |
| 29 | 96.4 | 1.3 | 95.6 | 3.1 |
| 30 | 94.3 | 0.6 | 94.8 | 4.6 |
| 31 | 92.4 | 0.9 | 94.3 | 4.8 |
| 32 | 93.2 | 0.5 | 93.9 | 4.6 |
| 33 | 90.3 | 1.2 | 91.9 | 6.9 |
| 34 | 90.4 | 1.3 | 89.4 | 9.3 |
| 35 | 91.2 | 1.8 | 89.3 | 8.9 |
| 36 | 90.2 | 0.5 | 89.0 | 10.5 |
| 37 | 89.5 | 0.0 | 85.9 | 14.1 |
| 38 | 89.4 | 1.2 | 87.6 | 11.2 |
| 39 | 96.7 | 0.4 | 88.0 | 11.6 |
| 40 | 87.5 | 0.9 | 89.4 | 9.7 |

Example 2

Purified pivalic acid was prepared in the same manner as in Example 1 except that amount of sodium hydroxide used was 6.0 g.

Substantially no sulfur matter (less than 1 ppm) was detected in the resulting purified pivalic acid and purity was 99.9% by weight. No carbonization of the residual oil was recognized.

Example 3

Purified pivalic acid was prepared in the same manner as in Example 1 except that 4.6 g of calcium hydroxide was used in place of 5.0 g of sodium hydroxide.

Sulfur content in the resulting purified pivalic acid was 3 ppm and purity was 99.7% by weight. No carbonization of the residual oil was recognized.

Example 4

Purified pivalic acid was prepared in the same manner as in Example 1 except that 15.5 g of sodium pivalate was used in place of 5.0 g of sodium hydroxide.

Sulfur content in the resulting purified pivalic acid was 1 ppm and purity was 99.9% by weight. No carbonization of the residual oil was recognized.

Comparative Example 1

Recovery operation for the complex sulfuric acid by washing of dichloromethane phase with water in Example 1 was repeated 3 times to obtain 3000 g of a solution of crude pivalic acid in dichloromethane. 673 ppm of sulfur was detected in said solution.

Dichloromethane was distilled off from this dichloromethane solution to obtain 300 g of crude pivalic acid.

This was distilled using the same Widmer column as used in Example 1.

The initial fraction (41.4 g) was a mixture of water, a small amount of polymerized oil and pivalic acid.

Then, 240 g of crude pivalic acid having sulfur content of 6380 ppm and having a purity of 99% by weight was obtained at 163-164°C.

The resulting purified pivalic acid was colored light yellow. The residual oil (34.5 g) was a mixture of pivalic acid, heavy oils, heavy acids, sulfates and sulfites and was colored black due to carbonization and further contained sludges. This residual oil was washed with water and pH of the washing solution was examoned by litmus paper to find that this was acidic.

Sulfur content in the residual oil was 14500 ppm.

## Claims

**1.** A process for producing a carboxylic acid which comprises a reaction step of reacting an olefin or alcohol, carbon monoxide and water in the presence of a catalyst containing sulfuric acid, an extraction step of extracting carboxylic acid in the reaction mixture obtained in said reaction step into an organic solvent, and a distillation step of distilling the organic phase obtained in said extraction step in the presence of at least one metal salt of carboxylic acid selected from the group consisting of an alkali metal salt of carboxylic acid and an alkaline earth metal salt of carboxylic acid, wherein the amount of the metal salt of carboxylic acid in the distillation step is more than the equivalent amount of sulfur compound present in the organic phase obtained in the extraction step.

**2.** A process according to claim 1 wherein the catalyst contains sulfuric acid in a concentration of 80-90% by weight.

**3.** A process according to claim 1 wherein the alkali metal in the alkali metal salt of carboxylic acid is sodium.

**4.** A process according to claim 1 wherein the alkaline earth metal in the alkaline earth metal salt of carboxylic acid is calcium.

**5.** A process according to claim 1 wherein the carboxylic acid in the metal salt of carboxylic acid is the same as contained in the reaction mixture.

**6.** A process according to claim 1 wherein the extraction step includes a step of contacting the reaction mixture obtained in the reaction step with an organic solvent to separate into an aqueous phase and an organic phase and a step of contacting the resulting organic phase with water in an amount of water which satisfies the following formula:

$$A(g) \leqq B \times (C/100) \times 18$$

wherein A represents amount of water to be added, B represents mol number of olefin used and C represents yield of carboxylic acid.

**7.** A process according to claim 6 wherein the organic solvent is a halogenated hydrocarbon.

**8.** A process according to claim 6 wherein the aqueous phase and the sulfuric acid extraction phase obtained in the extraction step are combined and used as the catalyst containing sulfuric acid in the reaction step.

## Revendications

**1.** Procédé de production d'acides carboxyliques, qui comprend une étape de réaction d'une oléfine ou d'un alcool, de monoxyde de carbone et d'eau en présence d'un catalyseur contenant de l'acide sulfurique, une étape d'extraction dans un solvant organique, de l'acide carboxylique présent dans le mélange réactionnel obtenu à l'issue de ladite étape de réaction, et une étape de distillation de la phase organique obtenue à l'issue de ladite étape d'extraction en présence d'au moins un sel métallique d'acide carboxylique, choisi parmi les sels de métal alcalin et alcalino-terreux d'acide carboxylique, dans lequel la quantité du sel métallique d'acide carboxylique dans l'étape de distillation surpasse, en quantité équivalente, celle de composé soufré présent dans la phase organique obtenue à l'étape d'extraction.

**2.** Procédé selon la revendication 1 dans lequel le catalyseur contient de l'acide sulfurique à une concentration de 80 à 90 % en poids.

**3.** Procédé selon la revendication 1 dans lequel le métal alcalin du sel de métal alcalin d'acide carboxylique est le sodium.

**4.** Procédé selon la revendication 1 dans lequel le métal alcalino-terreux du sel de métal alcalino-terreux d'acide carboxylique est le calcium.

**5.** Procédé selon la revendication 1 dans lequel l'acide carboxylique du sel métallique d'acide carboxylique est le même que celui contenu dans le mélange réactionnel.

**6.** Procédé selon la revendication 1 dans lequel l'étape d'extraction comprend une étape de mise en contact du mélange réactionnel obtenu à l'issue de l'étape de réaction avec un solvant organique, puis de séparation de la phase aqueuse et de la phase organique, et une étape de mise en contact de la phase organique obtenue avec une quantité d'eau satisfaisant la formule suivante :

$$A\ (g) \leqq B \times (C/100) \times 18$$

dans laquelle A représente la quantité d'eau à ajouter, B le nombre de moles d'oléfine utilisée et C le rendement en acide carboxylique.

**7.** Procédé selon la revendication 6, dans lequel le solvant organique est un hydrocarbure halogéné.

**8.** Procédé selon la revendication 6, dans lequel la phase aqueuse et la phase d'extraction contenant l'acide sulfurique, obtenues à l'issue de l'étape d'extraction, sont combinées et utilisées dans l'étape de réaction comme catalyseur contenant de l'acide sulfurique.

## Patentansprüche

**1.** Verfahren zur Herstellung einer Carbonsäure mit einem Reaktionsschritt der Umsetzung eines Olefins oder eines Alkohols mit Kohlenmonoxid und Wasser in Gegenwart eines Schwefelsäure enthaltenden Katalysators, einem Extraktionsschritt zur Extraktion der in der in dem Reaktionsschritt erhaltenen Reaktionsmischung enthaltenen Carbonsäure in ein organisches Lösungsmittel und einem Destillationsschritt zum Destillieren der in dem Extraktionsschritt erhaltenen organischen Phase in Gegenwart mindestens eines Metallsalzes einer Carbonsäure, ausgewählt aus der Gruppe, die aus Alkalimetallsalzen von Carbonsäuren und Erdalkalimetallsalzen von Carbonsäuren besteht, wobei die Menge des Metallsalzes der Carbonsäure in dem Destillationsschritt größer ist als die äquivalente Menge der Schwefelverbindung, die in der in dem Extraktionsschritt erhaltenen organischen Phase vorhanden ist.

**2.** Verfahren nach Anspruch 1, worin der Katalysator Schwefelsäure in einer Konzentration von 80 - 90 Gew.-% enthält.

**3.** Verfahren nach Anspruch 1, worin das Alkalimetall in dem Alkalimetallsalz der Carbonsäure Natrium ist.

**4.** Verfahren nach Anspruch 1, worin das Erdalkalimetall in dem Erdalkalimetallsalz der Carbonsäure Calcium ist.

**5.** Verfahren nach Anspruch 1, worin die Carbonsäure in dem Metallsalz der Carbonsäure die gleiche ist wie die in der Reaktionsmischung enthaltene.

**6.** Verfahren nach Anspruch 1, worin der Extraktionsschritt einen Schritt umfaßt, in dem die in dem Reaktionsschritt erhaltene Reaktionsmischung mit einem organischen Lösungsmittel in Kontakt gebracht wird, um sie in eine wäßrige Phase und eine organische Phase zu trennen, und einen Schritt, in dem die erhaltene organische Phase mit Wasser in Kontakt gebracht wird, in einer Menge des Wassers, welche die folgende Formel erfüllt:

$A(g) \leqq B \times (C/100) \times 18$

worin A für die Menge des zuzusetzenden Wassers, B für die Molzahl des verwendeten Olefins und C für die Ausbeute der Carbonsäure stehen.

**7.** Verfahren nach Anspruch 6, worin das organische Lösungsmittel ein halogenierter Kohlenwasserstoff ist.

**8.** Verfahren nach Anspruch 6, worin die wäßrige Phase und die in dem Extraktionsschritt erhaltene Schwefelsäure-Extraktionsphase vereinigt und als Schwefelsäure enthaltender Katalysator in dem Reaktionsschritt verwendet werden.